# EUROPEAN PATENT SPECIFICATION

(11) **EP 2 175 916 B1**
(45) Date of publication and mention of the grant of the patent: **09.09.2015**
(21) Application number: 08788235.3
(22) Date of filing: 28.07.2008
(51) Int. Cl.: A61M 5/20, A61M 5/32

(54) **INJECTION DEVICE**
INJEKTIONSVORRICHTUNG
DISPOSITIF D'INJECTION

(30) Priority: 08.08.2007 GB 0715459
(43) Date of publication of application: 21.04.2010
(73) Proprietor: Cilag GmbH International, 6300 Zug (CH)
(72) Inventor: JENNINGS, Douglas, Ivan, Royston, Hertfordshire SG8 7XU (GB)
(74) Representative: Tunstall, Christopher Stephen
(86) International application number: PCT/GB2008/002580
(87) International publication number: WO 2009/019439

(56) References cited:
- WO-A-2007/036676
- WO-A-2007/131013
- US-A- 5 997 513

## Description

### Field of the Invention

The present invention relates to an injection device of the type which has a syringe and which extends the syringe, discharges its contents and then retracts it automatically.

### Background of the Invention

Injection devices are shown in WO 95/35126 and EP-A-0 516 473. These devices employ a drive spring and some form of release mechanism that releases the syringe from the influence of the drive spring once its contents are supposed to have been discharged, to allow it to be retracted by a return spring.

Generally, the return spring is relatively weak, since its restoring force must be overcome by the drive spring, even while the drive spring is doing work on the various components of the injection device and the syringe during an injection cycle. This may give rise to a problem when the injection device is used with sealed hypodermic syringes, which typically have a hermetically sealed cover, needle shield or "boot" that covers the hypodermic needle and maintains the sterility of the syringe contents. Naturally, it is necessary to maintain the sterility of the syringe contents up to the point of administration, which devices that are designed to be disposable, as many will be, means that the boot must be removed with the syringe inside the injection device.

Typically, the action required to remove the boot from the syringe is simply to pull the boot away from the syringe, which requires a force in excess of 20N. This is significantly greater than the restoring force of the return spring, so the syringe will be pulled out of the injection device as the boot is removed and, when the boot comes away, it will snap back into place. This is not the best way to handle the syringe. The shock could damage it, the needle could be damaged and there may be problems re-engaging the syringe with those components of the injection device designed to act upon it. Even in cases where there is no return spring, for example where the syringe is held in place by friction with components of the injection device, the problem will still arise of relocating the syringe onto those components of the injection device designed to act upon it.

Moreover, there is a problem with having the syringe generally moveable in a direction out of the injection device. Accidental activation of the drive spring by mechanical failure of the drive spring's release mechanism (e.g. a trigger) can occur, for example by dropping the device on a hard surface. This accidental activation could cause the syringe to be extended unintentionally out of the device and its contents to be ejected. This could expose the needle of the syringe and increase the risk of inadvertent ski puncturing and/or infection.

WO2007/036676A discloses a injection device according to the preamble of claim 1 having a housing that receives a syringe, US 5997513 A discloses a syringe needle protection device and system for practising the same.

### Summary of the Invention

The injection device of the present invention is designed to deal with the aforementioned problems.

In a first aspect of the present invention, there is provided an injection device comprising:
a housing adapted to receive a syringe having a discharge nozzle, the syringe being movable along a longitudinal axis of the housing between a retracted position in which the discharge nozzle is contained within the housing and an extended position in which the discharge nozzle extends from the housing through an exit aperture;
a cap removably located over the exit aperture;
a syringe carrier adapted to support the syringe as it is advanced; and
a locking mechanism which is adapted, in an engaged position, to prevent removal of the cap from the housing and, in a disengaged position, to prevent movement of the syringe carrier towards the exit aperture relative to the housing but permit removal of the cap from the housing.

Thus, the syringe carrier and syringe are locked in place on disengagement of the locking mechanism which allows the cap to be removed whilst preventing the syringe being pulled forward as the cap is removed. The device can be actuated by disengaging the locking mechanism, removing the cap and reengaging the locking mechanism once the cap has been removed. This prevents damage to the syringe and its contents by ensuring that the syringe carrier and syringe cannot move when the cap is being removed.

Preferably, the locking mechanism comprises at least one locking component which moves between the engaged position and the disengaged position. More preferably, the injection device may comprise two locking components, wherein the locking components are disposed on opposite sides of the housing.

Each locking component may comprise a button which protrudes through an outer surface of the housing and each locking component is disengaged by applying pressure to its respective button. Preferably, the pressure is applied in a direction which not along the longitudinal axis.

In one embodiment of the present invention, each locking component comprises a resilient member which abuts the housing and maintains the locking mechanism in its engaged position when no pressure is applied to its respective button.

Each locking component may comprise a locking arm having a protrusion which engages with a ridge on the cap when the locking component is in its engaged position.

Advantageously, the housing of the injection device may comprise at least one resilient arm corresponding to each locking component, wherein each resilient arm is engageable with the syringe carrier, wherein the resilient arm is acted upon by its corresponding locking component in its disengaged position to engage with the syringe carrier, thereby preventing movement of the syringe carrier relative to the housing, wherein each locking component does not act upon its corresponding resilient arm when it is in its engaged position.

Preferably, each resilient arm comprises a protrusion and the syringe carrier comprises an aperture corresponding to each protrusion, wherein each protrusion engages with its corresponding aperture on the syringe carrier when its corresponding locking component is in its disengaged position to lock the syringe carrier to the housing.

Each protrusion may comprise a first abutment surface engageable with a corresponding second abutment surface on the edge of the aperture on the syringe carrier when its corresponding locking component is in its disengaged position and force is applied to the syringe carrier to move it towards the exit aperture along the longitudinal axis.

Preferably, the locking mechanism is located adjacent the exit aperture.

Preferably, the cap comprises a body and a sleeve located within the body and fixed relative to the body.

Preferably, a needle shield is removably located over the discharge nozzle,

Advantageously, the needle shield may be connected to the cap such that the needle shield is removed from the discharge nozzle during removal of the cap from the housing.

Moreover, the cap may comprise a shield retainer adapted to grip the needle shield, wherein the shield retainer is located within the sleeve.

In a second aspect of the present invention, there is provided a method of removing a cap from an injection device having a housing and a syringe located in the housing, the syringe moveable along a longitudinal axis of the housing between a retracted position in which the discharge nozzle is contained within the housing and an extended position in which the discharge nozzle extends from the housing through an exit aperture, the cap being located over the exit aperture and being connected to a needle shield of the syringe, the method comprising:
applying pressure to a surface of a button arranged on a locking component to move the locking component to a disengaged position so that it no longer engages the cap allowing it to be removed from the housing, the locking component engaging the cap in its engaged position to prevent its removal; and
moving the cap along the longitudinal axis away from the exit aperture such that the needle shield is moved along the longitudinal axis out of the exit aperture, thereby releasing it from the discharge nozzle whilst the locking component in its disengaged position prevents movement of the syringe along the longitudinal axis.

### Brief Description of the Drawings

The invention will now be described by way of example with reference to the accompanying drawings, in which:
Fig. 1a is a right-side view of the injection device according to the present invention;
Fig. 1b is a perspective view of the injection device of Fig. 1 with its cap removed;
Fig. 1c is a perspective view of the cap of the injection device of Fig. 1;
Fig. 2a is an exploded right-side view of the injection device of Fig. 1;
Fig. 2b is a right-side view of the assembled components of the injection device of Fig. 1;
Fig. 2c is a perspective view of a multi-component drive used in the injection device of Fig. 1
Fig. 3 is a cross-sectional view of the injection device of Fig. 1.

### Detailed Description of the Drawings

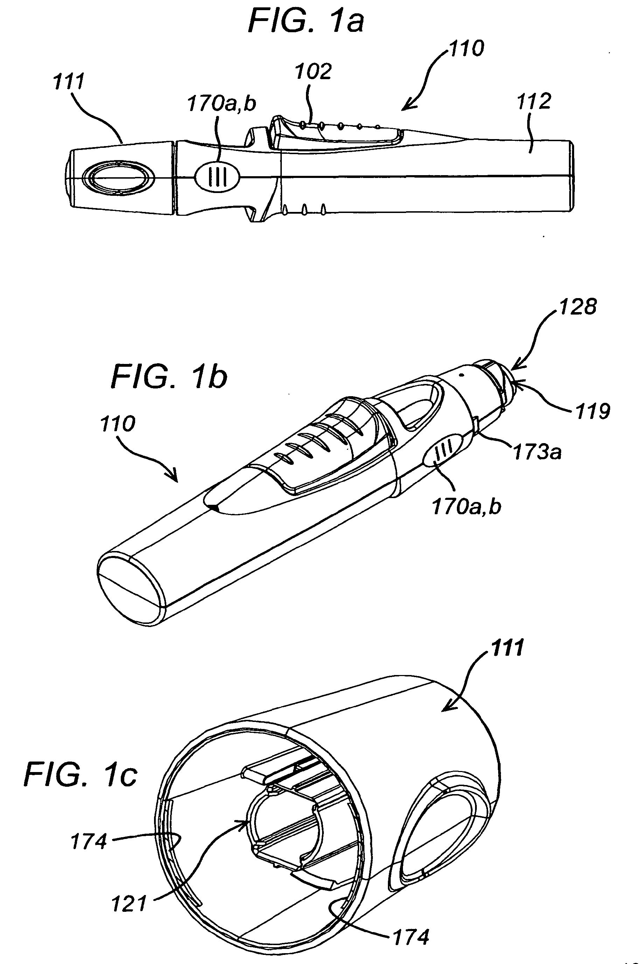

Fig. 1a is a right-side view of an injection device 110 according to the present invention.

The injection device 110 has a housing 112, a cap 111 which is removable from a proximal end 167 the housing 112 and a trigger button 102. Other parts of the device will be described in greater detail below. A locking component 170a, 170b is disposed on each of two sides of the housing 112 and protrudes through the housing.

Fig. 1b is a perspective view of the injection device 110 according to the present invention with the cap (not shown) removed from its end. The end of the housing 112 has an exit aperture 128, from which the end of a sleeve 119 can be seen to emerge.

Fig. 1c is a perspective view of the cap 111 of the injection device 110 according to the present invention. The cap 111 has a central boss 121 that fits within the sleeve 119 when the cap 111 is installed on the housing 112.

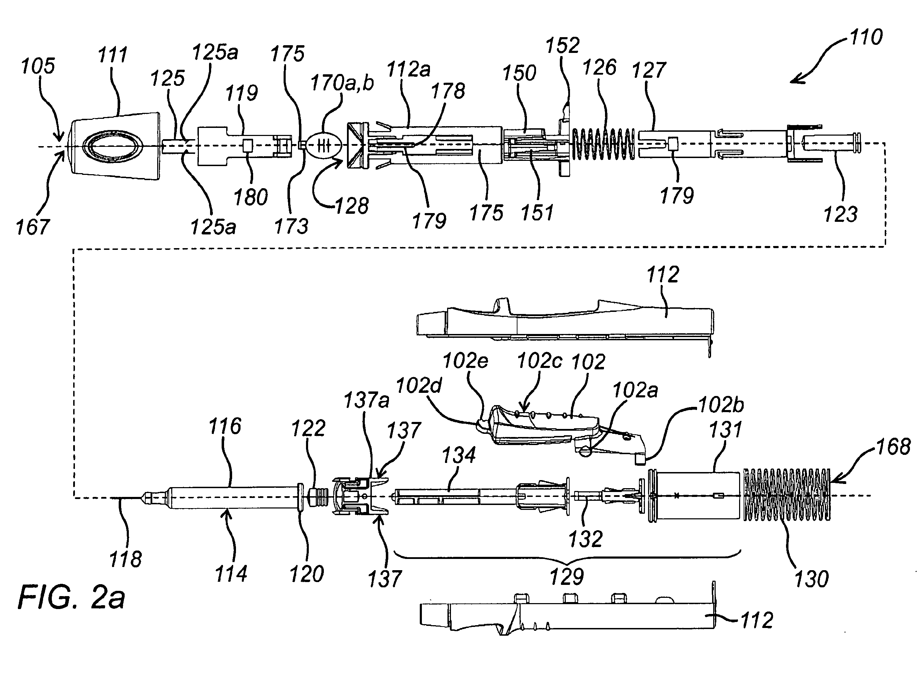

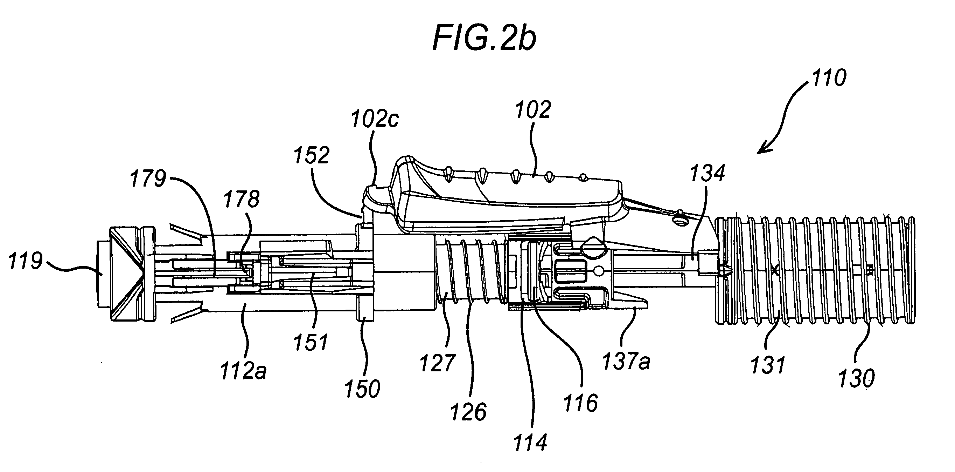

Fig. 2a is an exploded right-side view of the components of the injection device 110 according to the present invention and Fig. 2b is a right-side view of the assembled components of the injection device 110 according to the present invention without the housing 112 or cap 111.

As illustrated, the injection device 110 comprises a hypodermic syringe 114 of conventional type, including a syringe body 116 terminating at one end in a discharge nozzle, specifically a hypodermic needle 118, and at the other in a flange 120. The conventional plunger that would normally be used to discharge the contents of the syringe 114 manually has been removed and replaced with a drive element (referred to below as the second drive element 134) that contacts a bung 122 in the syringe 114. The bung 122 constrains a drug (not shown) to be administered within the syringe body 116. Whilst the syringe illustrated is of hypodermic type, this need not necessarily be so. Transcutaneous or ballistic dermal and subcutaneous syringes may also be used with the injection device of the present invention.

As illustrated, the injection device 110 includes a return spring 126 that biases the syringe 114 from an extended position in which the needle 118 extends from the aperture 128 in a case nose 112a of the housing 112 to a retracted position in which the needle 118 is contained within the housing 112. The return spring 126 acts on the syringe 114 via a syringe carrier 127. The syringe 114 is moveable along a longitudinal axis 105 of the injection device 110 which extends centrally along the length of the injection device 110 from the exit aperture 128 at its proximal end 167 to a distal end 168.

Contained within the housing at its distal end 168 is an actuator, which here takes the form of a compression drive spring 130. Drive from the drive spring 130 is transmitted via a multi-component drive 129 to the syringe 114 to advance it from its retracted position to its extended position and discharge its contents through the needle 118. The drive 129 accomplishes this task by acting directly on the drug and the syringe 114. Hydrostatic forces acting through the drug and, to a lesser extent, static friction between the bung 122 and the syringe body 116 initially ensure that they advance together, until the return spring 126 bottoms out on the syringe carrier 127 or meets some other obstruction (not shown) that retards its motion.

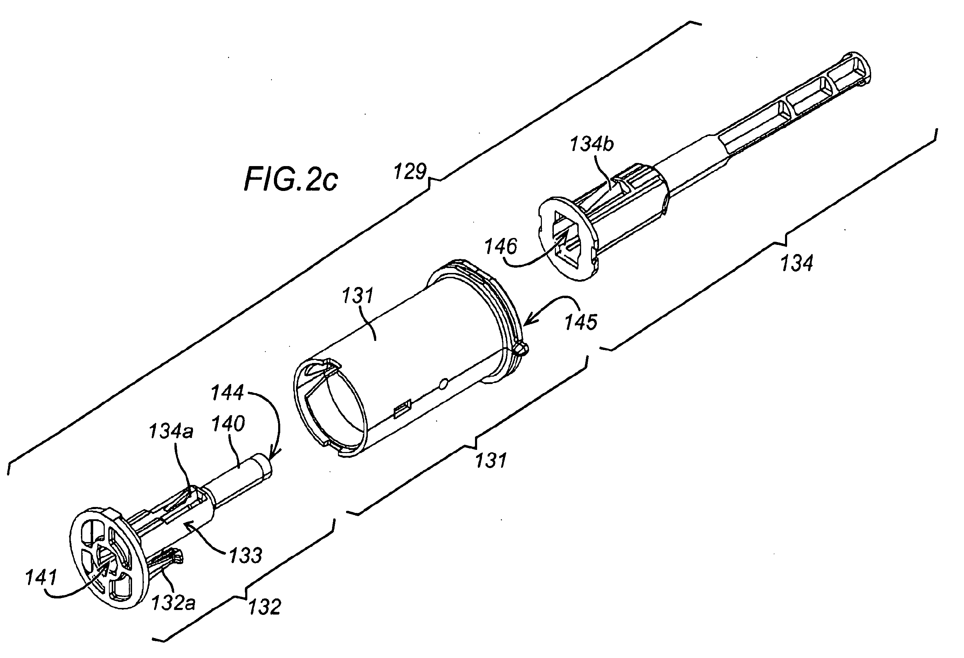

Fig. 2c is an exploded perspective view of the multi-component drive 129. The multi-component drive 129 between the drive spring 130 and the syringe 114 consists of three principal components. A drive sleeve 131 takes drive from the drive spring 130 and transmits it to a delay piston 133 on a first drive element 132. This in turn transmits drive to the second drive element 134.

As will be seen from Fig. 2c, the first drive element 132 includes a hollow stem 140, the inner cavity of which forms a collection chamber 141 in communication with a vent 144 that extends from the collection chamber 141 through the end of the stem 140. The second drive element 134 includes a blind bore 146 that is open at one end to receive the stem 140 and closed at the other. As will be appreciated, the bore 146 and the stem 140 define a fluid reservoir within which a damping fluid is contained.

The trigger button 102 is provided on the side of the housing 112 which, when in an engaged position with a proximal end 145 of the drive sleeve 131, serves to retain the drive spring 130 in a compressed state by contact between locking surface 102b and the drive sleeve 131 when the trigger button 102 is in an unactuated position. The trigger button 102 can pivot on the housing 112 via pivot 102a. When downwards pressure is applied to the trigger button 102 at an activation surface 102c (i.e. pressure directed into the housing 112), the locking surface 102b moves upwards in a direction away from the longitudinal axis 105. In this actuated position of the trigger button 102, the locking surface 102b is decoupled from the drive sleeve 131, thereby allowing the drive sleeve 131 to move relative to the housing 112 towards the exit aperture 128 under the influence of the drive spring 130.

The sliding sleeve 119 is moveable from its extended position (as shown in Fig. 1b) where it protrudes out of the exit aperture 128 into a retracted position in the case nose 112a of the housing 112. The sliding sleeve 119 is connected to a trigger button lock element 150 which has resilient arms 151 which bias the sliding sleeve 119 into its extended position in which its end protrudes from the end of the case nose 112a. Thus, application of pressure to the end of the sliding sleeve 119, for example by pressing the end of the sliding sleeve 119 against tissue, causes it to move into its retracted position into the housing 112; release of the pressure causes the sliding sleeve 119 to move into its extended position under bias from the resilient arms 151 acting against a side wall of the housing 112. The trigger button lock element 150 has a trigger button lock protrusion 152 which contacts with the end of a trigger button protrusion 102d on the trigger button 102 when the sliding sleeve is in its extended position. The trigger button protrusion 102 extends in a direction which is generally parallel to the longitudinal axis 105 of the injection device 110. The trigger button lock protrusion 152 extends in a direction which is generally perpendicular to the longitudinal axis 105 towards the trigger button protrusion 102d. The trigger button protrusion 102d has an aperture 102e which can move over the top of the trigger button lock protrusion 152 when the trigger button lock element 150 has been moved away from the exit aperture 128 (i.e. when the sliding sleeve 119 has been moved into the exit aperture 128 into its retracted position). In this position, the trigger button 102 can be moved into its deactivated position by rotating the trigger button 102 about the pivot 102a in the direction of the pressure applied to the pressure surface 102c. Thus, the trigger button lock element 150 and the sliding sleeve 119 act together to lock the trigger button 102 in its activated position (i.e. the locking surface 102b contacts the end of the drive sleeve 131 preventing it from moving towards the exit aperture 128 under the bias of the compressed drive spring 130).

When the sliding sleeve 119 has been moved into a position in which it is retracted into the housing 112 (i.e. into its unlocked position) and the trigger button 102 has been rotated into its deactivated position, the operation of the device 110 is then as follows.

Initially, the drive spring 130 moves the drive sleeve 131, the drive sleeve 131 moves the first drive element 132 and the first drive element 132 moves the second drive element 134, in each case by acting through flexible latch arms 132a, 134a, 134b. The second drive element 134 moves and, by virtue of static friction and hydrostatic forces acting through the drug (not shown), moves the syringe body 116 and syringe carrier 127 against the action of the return spring 126. The return spring 126 compresses and the hypodermic needle 118 emerges from the exit aperture 128 of the housing 112. This continues until the return spring 126 bottoms out or the syringe body 116 meets some other obstruction (not shown) that retards its motion. Because the static friction between the second drive element 134 and the syringe body 116 and the hydrostatic forces acting through the drug (not shown) to be administered are not sufficient to resist the full drive force developed by the drive spring 130, at this point the second drive element 134 begins to move within the syringe body 116 and the drug (not shown) begins to be discharged. Dynamic friction between the second drive element 134 and the syringe body 116 and hydrostatic forces acting through the drug (not shown) to be administered are, however, sufficient to retain the return spring 126 in its compressed state, so the hypodermic needle 118 remains extended.

Before the second drive element 134 reaches the end of its travel within the syringe body 116, so before the contents of the syringe have fully discharged, the flexible latch arms 134a, 134b linking the first and second drive elements 132, 134 reach a constriction 137 provided on a latch actuator element 137a which is fixed to the end of the syringe carrier 127. The constriction 137 moves the flexible latch arms 134a, 134b inwards from the position shown in Fig. 2c to a position at which the flexible latch arms 134a, 134b no longer couple the first drive element 132 to the second drive element 134, aided by the bevelled surfaces on the constriction 137. Once this happens, the first drive element 132 acts no longer on the second drive element 134, allowing the first drive element 132 to move relative to the second drive element 134.

Because the damping fluid is contained within a reservoir (not shown) defined between the end of the first drive element 132 and the blind bore 146 in the second drive element 134, the volume of the reservoir will tend to decrease as the first drive element 132 moves relative to the second drive element 134 when the former is acted upon by the drive spring 130. As the reservoir collapses, damping fluid is forced through the vent 144 into the collection chamber 141. Thus, once the flexible latch arms 134a, 134b have been released, the force exerted by the drive spring 130 does work on the damping fluid, causing it to flow though the constriction formed by the vent 144, and also acts hydrostatically through the fluid and through friction between the first and second drive elements 132, 134, thence via the second drive element 134. Losses associated with the flow of the damping fluid do not attenuate the force acting on the body of the syringe to a great extent. Thus, the return spring 126 remains compressed and the hypodermic needle remains extended.

After a time, the second drive element 134 completes its travel within the syringe body 116 and can go no further. At this point, the contents of the syringe 114 are completely discharged and the force exerted by the drive spring 130 acts to retain the second drive element 134 in its terminal position and to continue to cause the damping fluid to flow though the vent 144, allowing the first drive element 132 to continue its movement.

Before the reservoir of fluid is exhausted, the flexible latch arms 132a linking the drive sleeve 131 with the first drive element 132 reach another constriction (not shown) within the housing 112. This constriction moves the flexible latch arms 132a inwards from the position shown to a position at which they no longer couple the drive sleeve 131 to the first drive element 132, aided by bevelled surfaces on the constriction. Once this happens, the drive sleeve 131 acts no longer on the first drive element 132, allowing them to move relative each other. At this point, of course, the syringe 114 is released, because the forces developed by the drive spring 130 are no longer being transmitted to the syringe 114, and the only force acting on the syringe will be the return force from the return spring 126. Thus, the syringe 114 is now returned to its retracted position and the injection cycle is complete.

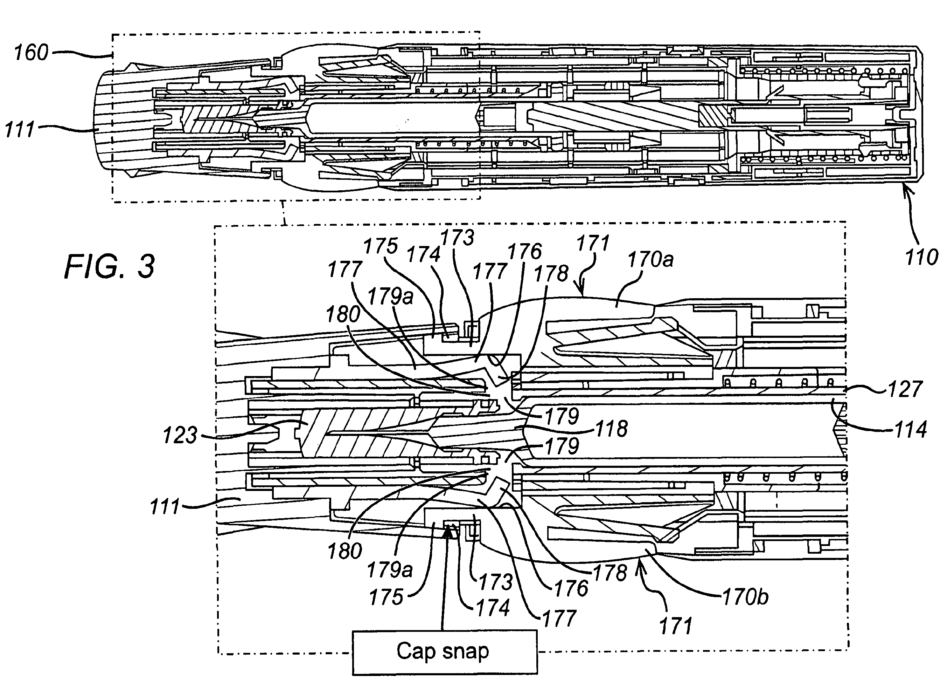

All this takes place, of course, only once the cap 111 has been removed from the end of the housing 112. The cap 111 can only be removed following disengagement of a locking mechanism 160, embodied in Fig. 2a by the locking component 170a, 170b. The locking component must be pressed inwardly into the injection device 110 (as will be explained further in conjunction with Fig. 3 below). The end of the syringe is sealed with a boot 123. The central boss 121 of the cap that fits within the sleeve 119 when the cap 111 is installed on the housing 112 comprises a retainer element 125 which is fixed into the boss 121. The retainer element 125 comprises resilient protrusions 125a which are directed away from the exit aperture 128. These resilient protrusions 125a deform as the cap 111 is inserted onto the housing 112 over a needle shield or rubber boot 123. The protrusions 125a then grip the boot 123 tightly so that the ends of the protrusions are slightly embedded in the boot 123 which might be made from rubber. This means that, as the cap 111 is pulled off the housing 112, the boot 123 is pulled away from the syringe 114 with the cap 111.

Fig. 2a also shows a syringe lock protrusion 170 located on the trigger button 102 at its distal end which is proximal to the end which is located nearest to the aperture 128. The syringe lock protrusion 170 extends in a generally perpendicular direction (with respect to the longitudinal axis 105) into the injection device 110 towards the longitudinal axis 105.

Fig. 3 shows how the locking mechanism 160 is integrated with the injection device 110 of the present invention.

The locking mechanism 160 comprises two locking components 170a, 170b which are disposed on opposite sides of the housing 112. Each locking component 170a, 170b comprises an activation surface 171 which protrudes through the exterior surface of the housing 112 in the form of a button.

Each locking component 170a, 170b is independently moveable from an engaged position (as shown in Fig. 3) to a disengaged position (not shown) by application of pressure to the activation surface 171.

Each locking component 170a, 170b also comprises a resilient member 172 which contacts a surface of the housing 112 at one end so that pressure applied to the activation surface 171 is resiliently opposed. When the pressure is released, the resilience in the member 172 causes the locking component 170a, 170b to move out of the housing 112 back into its engaged position.

In their engaged positions, each locking component 170a, 170b connects with the cap 111 via a locking arm 173 which has a flange 175 which engages with a ridge 174 on the cap 111. The locking arm 173 extends out of the housing 112 and sits between the case nose 112a and the housing 112.

Each locking component 170a, 170b has a pressure surface 176 which is located adjacent to a resilient arm 177 which is fixed onto or integrally formed with the housing 112. The resilient arm 177 comprises a protrusion 178 which is engageable with an aperture 179 on the syringe carrier 127. The outer surface of the protrusion 178 which is located towards the distal end 168 of the injection device 110 has a first abutment surface 178a. The edge of the aperture 179 that is located towards the distal end 168 of the injection device 110 forms a second abutment surface 179a. There is a further aperture 180 in the sliding sleeve 119 which permits the protrusion 178 to extend through it to engage with the aperture 179 on the syringe carrier 127. This means that the sliding sleeve 119 is locked in its extended position so that the trigger button 102 can not be activated (as explained above) during removal of the cap 111.

In the engaged position of the locking component, the resilient arm 177 is resiliently biased so that the protrusion 178 and aperture 179 are not engaged with each other. This means that the syringe carrier 127 could move towards the proximal end 167 of the injection device 110. However, the cap 111 is still located on the housing 112 and cannot be removed from the housing 112 because of the engagement of the flange 175 with the ridge 174.

When both locking components 170a, 170b are moved to their disengaged positions by application of force to the activation surfaces 171, the flange 175 and ridge 174 of each locking component 170a, 170b become disengaged from each other as a result of inward movement of the arm 173 towards the longitudinal axis 105, thereby permitting the cap 111 to be removed. However, since each locking component 170a, 170b has been moved inwardly with respect to the housing 112, the pressure surface 176 has moved inwardly towards the longitudinal axis 105 causing the protrusion 178 to move into the aperture 179. This means that the first and second abutment surfaces 178a, 179a will contact with each other if the syringe carrier 112 is moved towards the proximal end 167 of the injection device 110. This contact between the first and second abutment surfaces 178a, 179a prevents forward movement of the syringe carrier 127, for example when the cap 111 is being removed.

Thus, the syringe 114 and syringe carrier 127 are prevented from moving longitudinally until the cap 111 has been removed and that the locking mechanism 160 has returned to is engaged position (i.e. no pressure has been applied to the activation surfaces 171).

It will of course be understood that the present invention has been described above purely by way of example and modifications of detail can be made within the scope of the invention.

## Claims

1. An injection device (110) comprising:
a housing (112) adapted to receive a syringe (114) having a discharge nozzle (118), the syringe (114) being movable along a longitudinal axis of the housing (112) between a retracted position in which the discharge nozzle (118) is contained within the housing (112) and an extended position in which the discharge nozzle (118) extends from the housing (112) through an exit aperture (128),
a cap (111) removably located over the exit aperture (128)
a syringe carrier (127) adapted to support the syringe (114) as it is advanced; and **characterised by**
a locking mechanism (170a, 170b) which is adapted, in an engaged position, to prevent removal of the cap (111) from the housing (112) and, in a disengaged position, to prevent movement of the syringe carrier (127) towards the exit aperture (128) relative to the housing (112) but permit removal of the cap (111) from the housing (112).

2. The injection device according to claim 1, wherein the locking mechanism comprises at least one locking component which moves between the engaged position and the disengaged position.

3. The injection device according to claim 2, wherein the injection device comprises two locking components, wherein the locking components are disposed on opposite sides of the housing.

4. The injection device according to claim 2 or claim 3, wherein each locking component comprises a button which protrudes through an outer surface of the housing and each locking component is disengaged by applying pressure to its respective button.

5. The injection device according to claim 4, wherein the pressure is applied in a direction which not along the longitudinal axis.

6. The injection device according to claim 4 or claim 5, wherein each locking component comprises a resilient arm which abuts the housing and maintains the locking mechanism in its engaged position when no pressure is applied to its respective button.

7. The injection device according to any one of claims 2 to 6, wherein each locking component comprises a locking arm having a protrusion which engages with a ridge on the cap when the locking component is in its engaged position.

8. The injection device according to any one of claims 2 to 7, wherein housing of the injection device comprises at least one resilient arm corresponding to each locking component, wherein each resilient arm is engageable with the syringe carrier, wherein the resilient arm is acted upon by its corresponding locking component in its disengaged position to engage with the syringe carrier, thereby preventing movement of the syringe carrier relative to the housing, wherein each locking component does not act upon its corresponding resilient arm when it is in its engaged position.

9. The injection device of claim 9, wherein each resilient arm comprises a protrusion and the syringe carrier comprises an aperture corresponding to each protrusion, wherein each protrusion engages with its corresponding aperture on the syringe carrier when its corresponding locking component is in its disengaged position to lock the syringe carrier to the housing.

10. The injection device of claim 9, wherein each protrusion comprises a first abutment surface engageable with a corresponding second abutment surface on the edge of the aperture on the syringe carrier when its corresponding locking component is in its disengaged position and force is applied to the syringe carrier to move it towards the exit aperture along the longitudinal axis.

11. The injection device according to any one of the preceding claims, wherein the locking mechanism is located adjacent the exit aperture.

12. The injection device according to any one of the preceding claims, wherein the cap comprises a body and a sleeve located within the body and fixed relative to the body.

13. The injection device of any one of the preceding claims, further comprising a needle shield removably located over the discharge nozzle.

14. The injection device of claim 13, wherein the needle shield is connected to the cap such that the needle shield is removed from the discharge nozzle during removal of the cap from the housing.

15. The injection device according to claim 13 or claim 14 when dependant on claim 12, wherein the cap comprises a shield retainer adapted to grip the needle shield, wherein the shield retainer is located within the sleeve.

16. A method of removing a cap from an injection device having a housing and a syringe located in the housing, the syringe moveable along a longitudinal axis of the housing between a retracted position in which the discharge nozzle is contained within the housing and an extended position in which the discharge nozzle extends from the housing through an exit aperture, the cap being located over the exit aperture and being connected to a needle shield of the syringe, the method comprising:
applying pressure to a surface of a button arranged on a locking component to move the locking component to a disengaged position so that it no longer engages the cap allowing it to be removed from the housing, the locking component engaging the cap in its engaged position to prevent its removal; and
moving the cap along the longitudinal axis away from the exit aperture such that the needle shield is moved along the longitudinal axis out of the exit aperture, thereby releasing it from the discharge nozzle whilst the locking component in its disengaged position prevents movement of the syringe along the longitudinal axis.

## Patentansprüche

1. Injektionsvorrichtung (110), welche umfasst:
ein Gehäuse (112), das ausgebildet ist, eine Spritze (114) mit einer Austrittsdüse (118) aufzunehmen, wobei die Spritze (114) entlang einer Längsachse des Gehäuses (112) zwischen einer zurückgezogenen Stellung, in welcher die Austrittsdüse (118) im Gehäuse (112) enthalten ist, und einer ausgefahrenen Stellung, in welcher die Austrittsdüse (118) sich vom Gehäuse (112) durch eine Austrittsöffnung (128) erstreckt, bewegbar ist;
eine Kappe (111), die abnehmbar über der Austrittsöffnung (128) positioniert ist;
einen Spritzenträger (127), der ausgebildet ist, die Spritze (114) zu tragen, wenn sie vorwärts bewegt wird; und **gekennzeichnet durch**
einen Verriegelungsmechanismus (170a, 170b), welcher ausgebildet ist, in einer Eingriffsstellung das Abnehmen der Kappe (111) vom Gehäuse (112) zu verhindern und in einer Außereingriffsstellung die Bewegung des Spritzenträgers (127) zur Austrittsöffnung (128) hin relativ zum Gehäuse (112) zu verhindern, aber das Abnehmen der Kappe (111) vom Gehäuse (112) zu erlauben.

2. Injektionsvorrichtung nach Anspruch 1, wobei der Verriegelungsmechanismus mindestens eine Verriegelungskomponente umfasst, welche sich zwischen der Eingriffsstellung und der Außereingriffsstellung bewegt.

3. Injektionsvorrichtung nach Anspruch 2, wobei die Injektionsvorrichtung zwei Verriegelungskomponenten umfasst, wobei die Verriegelungskomponenten auf entgegengesetzten Seiten des Gehäuses angeordnet sind.

4. Injektionsvorrichtung nach Anspruch 2 oder Anspruch 3, wobei jede Verriegelungskomponente einen Knopf umfasst, welcher durch eine Außenfläche des Gehäuses hervorsteht, und wobei jede Verriegelungskomponente durch Aufbringen von Druck auf ihren jeweiligen Knopf außer Eingriff gebracht wird.

5. Injektionsvorrichtung nach Anspruch 4, wobei der Druck in einer Richtung nicht entlang der Längsachse aufgebracht wird.

6. Injektionsvorrichtung nach Anspruch 4 oder Anspruch 5, wobei jede Verriegelungskomponente einen elastischen Arm umfasst, welcher am Gehäuse anliegt und den Verriegelungsmechanismus in seiner Eingriffsstellung hält, wenn kein Druck auf ihren jeweiligen Knopf aufgebracht wird.

7. Injektionsvorrichtung nach einem der Ansprüche 2 bis 6, wobei jede Verriegelungskomponente einen Verriegelungsarm mit einem Vorsprung umfasst, welcher mit einem Wulst auf der Kappe in Eingriff ist, wenn die Verriegelungskomponente sich in ihrer Eingriffsstellung befindet.

8. Injektionsvorrichtung nach einem der Ansprüche 2 to 7, wobei das Gehäuse der Injektionsvorrichtung mindestens einen elastischen Arm entsprechend jeder Verriegelungskomponente umfasst, wobei jeder elastische Arm mit dem Spritzenträger in Eingriff bringbar ist, wobei jede Verriegelungskomponente in ihrer Außereingriffsstellung auf ihren entsprechenden elastischen Arm einwirkt, mit dem Spritzenträger in Eingriff zu kommen, wodurch eine Bewegung des Spritzenträgers relativ zum Gehäuse vermieden wird, wobei jede Verriegelungskomponente nicht auf ihren entsprechenden elastischen Arm einwirkt, wenn sie in ihrer Eingriffsstellung ist.

9. Injektionsvorrichtung nach Anspruch 9, wobei jeder elastische Arm einen Vorsprung umfasst, und der Spritzenträger eine Öffnung entsprechend jedem Vorsprung umfasst, wobei jeder Vorsprung mit seiner entsprechenden Öffnung auf dem Spritzenträger in Eingriff kommt, wenn seine entsprechende Verriegelungskomponente in ihrer Außereingriffsstellung ist, um den Spritzenträger am Gehäuse zu verriegeln.

10. Injektionsvorrichtung nach Anspruch 9, wobei jeder Vorsprung eine erste Anlagefläche umfasst, die mit einer entsprechenden zweiten Anlagefläche auf der Kante der Öffnung am Spritzenträger in Eingriff bringbar ist, wenn seine entsprechende Verriegelungskomponente in ihrer Außereingriffsstellung ist, und eine Kraft auf den Spritzenträger aufgebracht wird, um ihn entlang der Längsachse zur Austrittsöffnung hin zu bewegen.

11. Injektionsvorrichtung nach einem der vorhergehenden Ansprüche, wobei der Verriegelungsmechanismus sich angrenzend zur Austrittsöffnung befindet.

12. Injektionsvorrichtung nach einem der vorhergehenden Ansprüche, wobei die Kappe einen Körper und eine Hülse umfasst, die sich innerhalb des Körpers befindet und relativ zum Körper fixiert ist.

13. Injektionsvorrichtung nach einem der vorhergehenden Ansprüche, welche ferner einen Nadelschutz umfasst, der abnehmbar über der Austrittsdüse angeordnet ist.

14. Injektionsvorrichtung nach Anspruch 13, wobei der Nadelschutz mit der Kappe derart verbunden ist, dass der Nadelschutz von der Austrittsdüse abgenommen wird, wenn die Kappe vom Gehäuse abgenommen wird.

15. Injektionsvorrichtung nach Anspruch 13 oder Anspruch 14, falls abhängig von Anspruch 12, wobei die Kappe einen Schutzhalter umfasst, der ausgebildet ist, den Nadelschutz zu erfassen, wobei der Schutzhalter innerhalb der Hülse positioniert ist.

16. Verfahren zum Abnehmen einer Kappe von einer Injektionsvorrichtung mit einem Gehäuse und einer im Gehäuse angeordneten Spritze, wobei die Spritze entlang einer Längsachse des Gehäuses zwischen einer zurückgezogenen Stellung, in welcher die Austrittsdüse im Gehäuse enthalten ist, und einer ausgefahrenen Stellung, in welcher die Austrittsdüse sich vom Gehäuse durch eine Austrittsöffnung erstreckt, bewegbar ist, wobei die Kappe über der Austrittsöffnung positioniert ist und mit einem Nadelschutz der Spritze verbunden ist, wobei das Verfahren umfasst:
Aufbringen von Druck auf eine Fläche eines Knopfs, der auf einer Verriegelungskomponente angeordnet ist, um die Verriegelungskomponente in eine Außereingriffsstellung zu bewegen, so dass sie nicht mehr mit der Kappe in Eingriff ist und diese vom Gehäuse abgenommen werden kann, wobei die Verriegelungskomponente in ihrer Eingriffsstellung mit der Kappe in Eingriff ist, um ihr Abnehmen zu verhindern; und
Bewegen der Kappe entlang der Längsachse von der Austrittsöffnung weg, so dass der Nadelschutz entlang der Längsachse aus der Austrittsöffnung bewegt wird, wodurch sie von der der Austrittsdüse gelöst wird, während die Verriegelungskomponente in ihrer Außereingriffsstellung die Bewegung der Spritze entlang der Längsachse verhindert.

## Revendications

1. Dispositif (110) d'injection comportant :
un logement (112) conçu pour recevoir une seringue (114) présentant une buse (118) d'évacuation, la seringue (114) étant mobile le long d'un axe longitudinal du logement (112) entre une position rentrée dans laquelle la buse (118) d'évacuation est continue à l'intérieur du logement (112) et une position sortie dans laquelle la buse (118) d'évacuation s'étend du logement (112) par une ouverture (128) de sortie ;
un capuchon (111) situé de manière amovible sur l'ouverture (128) de sortie ;
un porte-seringue (127) conçu pour supporter la seringue (114) au fur et à mesure que cette dernière est avancée ; et **caractérisé par** un
un mécanisme (170a, 170b) de verrouillage qui est conçu, dans une position en prise, pour empêcher l'enlèvement du capuchon (111) du logement (112) et, dans une position dégagée, pour empêcher le déplacement du porte-seringue (127) vers l'ouverture (128) de sortie par rapport au logement (112) mais pour permettre l'enlèvement du capuchon (111) du logement (112).

2. Dispositif d'injection selon la revendication 1, le mécanisme de verrouillage comportant au moins un composant de verrouillage qui se déplace entre la position en prise et la position dégagée.

3. Dispositif d'injection selon la revendication 2, le dispositif d'injection comportant deux composants de verrouillage, les composants de verrouillage étant disposés sur des côtés opposés du logement.

4. Dispositif d'injection selon la revendication 2 ou la revendication 3, chaque composant de verrouillage comportant un bouton qui fait saillie à travers une surface externe du logement et chaque composant de verrouillage est dégagé en appliquant une pression sur son bouton respectif.

5. Dispositif d'injection selon la revendication 4, la pression étant appliquée dans une direction qui n'est pas le long de l'axe longitudinal.

6. Dispositif d'injection selon la revendication 4 ou la revendication 5, chaque composant de verrouillage comportant un bras élastique qui vient buter contre le logement et maintient le mécanisme de verrouillage dans sa position en prise lorsqu'il n'y a pas de pression appliquée sur son bouton respectif.

7. Dispositif d'injection selon l'une quelconque des revendications 2 à 6, chaque composant de verrouillage comportant un bras de verrouillage présentant une protubérance qui vient en prise avec une crête sur le capuchon lorsque le composant de verrouillage est dans sa position en prise.

8. Dispositif d'injection selon l'une quelconque des revendications 2 à 7, le logement du dispositif d'injection comportant au moins un bras élastique correspondant à chaque composant de verrouillage, chaque bras élastique pouvant venir en prise avec le porte-seringue, le bras élastique pouvant être sollicité par son composant de verrouillage correspondant dans sa position dégagée pour se mettre en prise avec le porte-seringue, empêchant ainsi le déplacement du porte-seringue par rapport au logement, chaque composant de verrouillage ne sollicitant pas son bras élastique correspondant lorsqu'il est dans sa position en prise.

9. Dispositif d'injection selon la revendication 9, chaque bras élastique comportant une protubérance et le porte-seringue comportant une ouverture correspondant à chaque protubérance, chaque protubérance venant en prise avec son ouverture correspondante sur le porte-seringue lorsque son composant de verrouillage correspondant est dans sa position dégagée pour verrouiller le porte-seringue sur le logement.

10. Dispositif d'injection selon la revendication 9, chaque protubérance comportant une première surface venant en butée pouvant venir en prise avec une seconde surface venant en butée correspondante sur le bord de l'ouverture du porte-seringue lorsque son composant de verrouillage correspondant est dans sa position dégagée et une force est appliquée au porte-seringue pour le déplacer vers l'ouverture de sortie le long de l'axe longitudinal.

11. Dispositif d'injection selon l'une quelconque des revendications précédentes, le mécanisme de verrouillage étant situé à côté de l'ouverture de sortie.

12. Dispositif d'injection selon l'une quelconque des revendications précédentes, le capuchon comportant un corps et un manchon situé à l'intérieur du corps et fixé par rapport au corps.

13. Dispositif d'injection selon l'une quelconque des revendications précédentes, comportant en outre un protecteur d'aiguille situé de manière amovible sur la buse d'évacuation.

14. Dispositif d'injection selon la revendication 13, le protecteur d'aiguille étant relié au capuchon de telle sorte que le protecteur d'aiguille est enlevé de la buse d'évacuation au moment où le capuchon est enlevé du logement.

15. Dispositif d'injection selon la revendication 13 ou la revendication 14 lorsque dépendante de la revendication 12, le capuchon comportant un dispositif de retenue de protecteur conçu pour saisir le protecteur d'aiguille, le dispositif de retenue de protecteur étant situé à l'intérieur du manchon.

16. Procédé d'enlèvement d'un capuchon d'un dispositif d'injection muni d'un logement et d'une seringue située dans le logement, la seringue étant mobile le long d'un axe longitudinal du logement entre une position rentrée dans laquelle la buse d'évacuation est contenue à l'intérieur du logement et une position sortie dans laquelle la buse d'évacuation s'étend du logement par une ouverture de sortie, le capuchon étant situé sur l'ouverture de sortie et étant relié à un protecteur d'aiguille de la seringue, le procédé consistant à :
appliquer une pression sur une surface d'un bouton disposé sur un composant de verrouillage pour déplacer le composant de verrouillage vers une position dégagée de façon à ce qu'il ne soit plus en prise avec le capuchon et permette à ce dernier d'être enlevé du logement, le composant de verrouillage mettant en prise le capuchon dans sa position en prise pour empêcher son enlèvement ; et
déplacer le capuchon le long de l'axe longitudinal à distance de l'ouverture de sortie de telle sorte que le protecteur d'aiguille est déplacé le long de l'axe longitudinal hors de l'ouverture de sortie, ce qui le libère de la buse d'évacuation alors que le composant de verrouillage dans sa position dégagée empêche le déplacement de la seringue le long de l'axe longitudinal.
